(19) Europäisches Patentamt
European Patent Office
Office européen des brevets

(11) Publication number: **0 170 830**
**B1**

# (12) EUROPEAN PATENT SPECIFICATION

(45) Date of publication of patent specification: **22.03.89**

(51) Int. Cl.[4]: **C 07 C 103/48**, C 07 C 102/00

(21) Application number: **85107302.3**

(22) Date of filing: **13.06.85**

## (54) A process for amido carbonylation of an aldehyde to n-acetyl alpha-amino acids.

(30) Priority: **27.07.84 US 635077**

(43) Date of publication of application:
**12.02.86 Bulletin 86/07**

(45) Publication of the grant of the patent:
**22.03.89 Bulletin 89/12**

(84) Designated Contracting States:
**BE CH DE FR GB IT LI NL**

(56) References cited:
**EP-A-0 041 587**
**FR-A-2 089 136**
**FR-A-2 516 508**
**FR-A-2 523 576**

(73) Proprietor: **TEXACO DEVELOPMENT CORPORATION**
**2000 Westchester Avenue**
**White Plains New York 10650 (US)**

(72) Inventor: **Lin, Jiang-Jen**
**2617 Oak Meadow Drive**
**Round Rock Texas 78761 (US)**
Inventor: **Knifton, John Frederick**
**10900 Catskill Trail**
**Austin Texas 78750 (US)**
Inventor: **Yeakey, Ernest Leon**
**6316 Gato Path**
**Austin Texas 78731 (US)**

(74) Representative: **Schupfner, Gerhard D.**
**Müller, Schupfner & Gauger Karlstrasse 5**
**Postfach 14 27**
**D-2110 Buchholz/Nordheide (DE)**

**EP 0 170 830 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Field of the Invention

This invention relates to the catalytic amido carbonylation of aldehydes in the presence of amides which leads to N-acyl-α-amino acids.

More particularly this invention leads to an improved process for production of N-acetylglycine, an intermediate for glycine production by hydrolysis, wherein paraformaldehyde is reacted with an amide and CO in the presence of a cobalt-containing compound and a novel promoter at a temperature of at least 25°C and a pressure of at least 34 bar.

Background of the Invention

Transition metal carbonyl catalyzed carbonylations of olefins, halides and alchols have been extensively studied for a long time because of their importance both as laboratory methods and as industrial processes.

One of the most interesting reactions using carbon monoxide is the cobalt-catalyzed amidocarbonylation of aldehydes which leads to the formation of a variety of N-acyl- -amino acids, including N-acetylglycine.

This reaction was first reported by Wakamatsu, in Chemical Communication, 1540, (1971) and U.S. Patent No. 3,766,266, where an aldehyde, amide and carbon monoxide were reacted together in the presence of dicobalt octacarbonyl catalyst to give N-acyl-α-amino acid.

An article by Pino, et al., in *Journal of Molecular Catalysis,* 6 (1979) 341—350, discusses the synthesis potential and the catalytic mechanism for the reaction wherein N-acyl-α-amino acids are produced by reacting an aldehyde, CO and amide in the presence of dicobalt octacarbonyl.

In *Synthetic Communications,* 12 (14) 1111—1113 (1982). Stern, et al. discuss a process for obtaining N-acylimino diacetic acid by basically the same process first discovered by Wakamastsu and later investigated by Pino for obtaining good yield of N-acyl-α-amino acid. This study demonstrated that the use of a controlled amount of water in the reaction mixture is the important factor for N-acylimino diacetic acid synthesis.

In amidocarbonylation, the aldehyde can be generated in situ from allyl alcohol, oxiranes, alcohols and olefins then followed by the reaction with amide and carbon monoxide to produce N-acyl-α-amino acid.

Hirai, et al. discuss a process for combining the transition metal catalyzed isomerization of allyl alcohol to aldehyde and cobalt catalyzed amidocarbonylation to provide a route from allylic alcohols to N-acyl-α-amino acids. See *Tetrahedron Letters,* Vol. 23, No. 24, pp. 2491—2494, 1982.

In German Offen. DE 3,242,374 or U.K. Patent Application GB 2111982A oxiranes are reacted with amides in the presence of CO and $H_2$, a cobalt-containing catalyst and a Group I—IV metal containing compound as promoter to yield N-acetylphenylalanine.

In U.S. Patent No. 3,996,288 a method is disclosed for reacting a hydrochloric or hydrobromic acid ester in the presence of hydrogen carbon monoxide and the amide of a carboxylic acid and a carbonylation catalyst to produce an aldehyde having one or more carbon atoms than the alcohol or ester. This aldehyde can be further reacted to form an N-acylamino acid.

U.S. Patent No. 4,264,515 discloses a process for obtaining terminal N-acyl-α-amino acids by a reaction catalyzed by a cobalt carbonylation catalyst wherein the aldehyde is produced in situ from olefins and $CO/H_2$ mixtures.

Among these disclosures, dicobalt octacarbonyl was generally used as the active catalyst for amidocarbonylation. In the case of the paraformaldehyde, acetamide and carbon monoxide reaction, N-acetyl glycine was the major product. This glycine derivative is highly polar and can act as a strong ligand by chelating with the cobalt catalyst. Therefore, the separation of cobalt catalyst and solid N-acetylglycine product has been difficult. Furthermore, the product selectivity between N-acetylglycine and bis-amidomethane has not been defined.

Our disclosures preferably involved the use of a novel ligand-sulfoxide compound, complexing with dicobalt octacarbonyl, which catalyzes the amidocarbonylation of paraformaldehyde in high selectivity and with easy separation of cobalt catalyst and N-acetylglycine product.

Summary of the Invention

This invention concerns a method for making N-acyl-α-amino acids as exemplified by N-acetylglycine which comprises contacting a mixture of an aldehyde such as paraformaldehyde and carbon monoxide, hydrogen and an amide with a catalyst comprising a cobalt-containing compound promoted by a ligand containing one or more sulfoxide groups in a substantially inert solvent at a pressure of at least 34 bar and a temperature of at least 25°C.

Detailed Description of the Invention

In the narrower and more preferred practice of this invention N-acylglycines are prepared from a mixture of paraformaldehyde, an amide, carbon monoxide and hydrogen by a process which comprises contacting said mixture with a catalyst system comprising a cobalt-containing compound promoted by a ligand containing one or more sulfoxide groups which is dissolved in a substantially inert solvent at a

temperature of at least 25°C and a pressure of at least 34 bar until substantial formation of the desired N-acetylglycine has been achieved.

The reaction can best be represented by the following Equation 1;

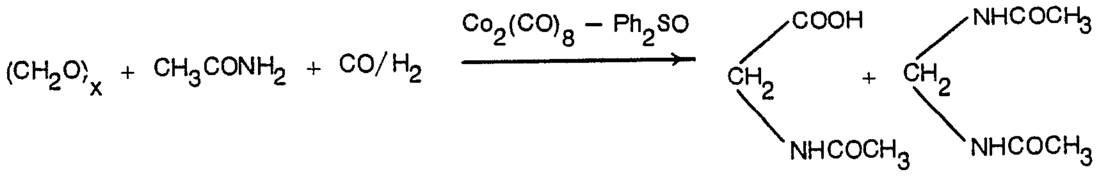

(1)

Recovery of N-acetylglycine from the reaction product can be carried out in any convenient or conventional manner such as by distillation, extraction, filtration, crystallization, etc.

The catalyst system suitable for the practice of this invention comprises a cobalt-containing compound in a substantially inert solvent promoted by a ligand containing one or more sulfoxide groups.

In the catalyst system of this invention the cobalt-containing compound and the ligand-containing one or more sulfoxide groups are believed to be in complex equilibrium during amidocarbonylation in such a way that this catalyst system provides two important advantages over the use of cobalt alone:

1) It gives higher yields and selectivities of N-acetyl amino acid product than can be obtained with a catalyst which utilizes solely a cobalt-containing compound dispersed in a solvent.

2) There is ease of recovery of the cobalt, in solution, from the solid N-acetylamido acid product (e.g. N-acetylglycine). The N-acetylglycine may then be converted to glycine by hydrolysis.

The cobalt-containing compound may take many different forms. For instance, the cobalt may be added to the reaction mixture in the form of a variety of inorganic or organic cobalt salts, or cobalt carbonyls. The cobalt may, for example, be added as a cobalt halide such as cobalt bromide or cobalt chloride, or it may be added as the salt of an aliphatic or aromatic carboxylic acid such as, for example, cobalt formate, cobalt acetate, cobalt butyrate, cobalt naphenate and cobalt stearate. The cobalt carbonyl may be tetracobalt dodecacarbonyl or dicobalt octacarbonyl. The preferred cobalt-containing compound is dicobalt octacarbonyl.

The promoter to be used in this catalyst system may contain one or more sulfoxide groups per molecule.

The general structure of sulfoxide promoter can be described as follows:

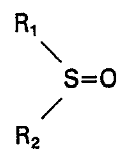

The $R_1$ and $R_2$ group can be alkyls, such as methyl, ethyl, n-butyl or n-hexyl, aromatic groups such as phenyl, chlorophenyl, aminophenyl or tolyl, arylalkyls such as benzyl or chlorobenzyl. The different $R_1$ and $R_2$ groups can also exist in the same sulfoxide molecule. Suitable examples include methyl sulfoxide, ethyl sulfoxide, n-butyl sulfoxide, methyl n-butyl sulfoxide, diphenyl sulfoxide, benzyl sulfoxide, methyl phenyl sulfoxide, 4-chlorophenyl sulfoxide and p-tolyl sulfoxide. The cyclic sulfoxide can also be used; such as tetramethylene sulfoxide.

Various aldehydes may be used as feedstock in the method of this invention. More specifically aliphatic, alicyclic, aromatic and heterocyclic aldehydes have been used successfully in the method of the invention. Aldehydes giving good yields with suitable amides include paraformaldehyde, formaldehyde, trioxane, acetaldehyde, propionaldehyde, butyraldehyde, phenylacetylaldehyde, 2,4-dihydroxyphenylacetaldehyde, indolylacetaldehyde, crotonaldehyde, β-formylpropionaldehyde, β-formylpropionic acid and its esters, β-methylmercaptopropionaldehyde, glycolaldehyde, α-acetoxy-propionaldehyde, stearaldehyde, benzaldehyde, furfural, indolaldehyde, adipaldehyde and acrolein. Most preferred are paraformaldehyde and formaldehyde.

Suitable amid-containing coreactants that are useful in the amidocarbonylation reaction have the general structure:

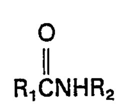

where the $R_1$ and $R_2$ groups may be a combination of aryl, alkyl, arylalkyl and alkylaryl hydrocarbonyl radicals, or hydrogen, including the methyl, ethyl, butyl, n-octyl, phenyl, benzyl and chlorophenyl groupings. Examples of suitable amide coreactants include acetamide, benzamide, formamide, N-methylformamide, lauramide and n-methylbenzamide.

The quantity of cobalt-containing compound and sulfoxide-containing promoter to be used in the process of the invention may vary. The process is conducted in the presence of a catalytically effective quantity of the active cobalt-containing compound and the active sulfoxide-containing promoter which gives the desired product in reasonable yield. The reaction proceeds when employing as little a 0.1 weight percent, and even lesser amounts of the cobalt-containing compound, along with as little as 0.1 weight percent of the sulfoxide-containing promoter based on the total weight of the reaction mixture. The upper concentration is dictated by a variety of factors including catalyst cost, partial pressures of carbon monoxide and hydrogen and operating temperature. A cobalt-containing compound concentration of from 0.1 to 10 weight percent in conjunction with a sulfoxide-containing compound concentration of from 0.1 to 10 percent, based on the total weight of the reaction mixture is generally desirable in the practice of this invention.

Particularly superior results are obtained when the above-noted components of the catalyst system are combined as follows on a molar basis: Cobalt-containing compound to sufoxide-containing promoter, 1.0:0.1 to 1.0:5.0.

As characterized above, this process is operated as a homogeneous liquid phase mixture. The reaction is operated in an inert solvent. Preferred inert solvents are those which permit as least partial dissolution of the cobalt catalyst precursor, the amide and the aldehyde compound. These are generally polar solvents, for example of the ester, ether, ketone, amide, sulfoxide or aromatic hydrocarbon type.

Methyl and ethyl acetate are examples of suitable solvents. Other polar solvents are ethers, such as p-dioxane, methyl tertiary butyl ether, methyl tertiary amyl ether or tetrahydrofuran, tertiary amides, such as dimethyl formamide, dimethyl sulfoxide and ethylene carbonate.

The preferred solvent is ethyl acetate.

The N-acylamino acids are often insoluble in the solvent phase. This permits separation of the cobalt catalyst which may dissolve into the solvent phase, with or without prior acidification.

The operating conditions may vary over a wide range. The reaction temperature may vary from 25°C to 300°C. The preferred temperature is from 80°C to 150°C. The pressure may range from 34 bar to 276 bar or more. It appears that higher selectivities are obtained when operating at moderate pressures, in the range from 69 to 241 bar.

The amidocarbonylation reaction of this invention is best conducted in a carbon monoxide-rich atmosphere, although some hydrogen gas should also be present in order to achieve maximum cobalt catalyst activity. The hydrogen to cabon monoxide molar ratio in the reactor may be varied, for example, within the range from 20:1 to 1:20, but preferably it should be rich in carbon monoxide and the $H_2$:CO ratio should be in the range 1:1 to 1:5.

The carbon monoxide employed need not satisfy particular purity requirements although catalyst contaminants should be avoided if the reaction is intended to continue over an extended period. Particularly in continuous operations, but also in batch experiments, the carbon monoxide and hydrogen gas may also be used in conjunction with up to 10% by volume of one or more other gases. These other gases may include one or more inert gases such as argon and nitrogen or they may include gases that may, or may not, undergo reaction under carbon monoxide hydrogenation conditions, such as carbon dioxide and hydrocarbons, such as methane, ethane and propane, ethers, such as dimethyl ether, methyl ethyl ether and diethyl ether and alkanols, such as methanol.

In all these synthesis in order to achieve a high degree of selectivity the amount of carbon monoxide, aldehyde and amide present in the reaction mixture should be sufficient to at least satisfy the stoichiometry of the desired formation of N-acyl-α-amino acids as shown in Equation I above. Excess carbon monoxide over the stoichiometric amount may be present and is desirable.

The preferred products of this synthesis are N-acylaminoacids. The main desired product, N-acetylglycine, will be formed in significant quantities. Also formed are significant amounts of bisamidal, a condensation product of paraformaldehyde and acetamide. Each of these products, including by-products can be recovered from the reaction mixture by conventional means, e.g. recrystallization.

The novel process of this invention can be conducted in a batch, semi-continuous or continuous manner. The catalyst can be initially introduced into the reaction zone batchwise, or it may be continuously or intermittently introduced into such a zone during the course of the synthesis reaction. Operating conditions can be adjusted to optimize the formation of the desired amino acid product, and said material may be recovered by methods known to the art, such as filtration, recrystallization, distillation and extraction. A fraction rich in the catalyst components may then be recycled to the reaction zone, if desired, and additional products generated.

The products have been identified in this work by one or more of the following analytical procedures: viz, gas-liquid phase chromatography (glc), gas chromatography/infrared spectroscopy (GC/IR), nuclear magnetic resonance (nmr) and elemental analysis, or a combination of these techniques. Analysis have for the most part, been by molar weight; all temperatures are in degrees centigrade and all pressures in bar.

The yield of N-acetylglycine in each synthesis (mole %) is estimated basis equation 1 using the formula:

$$\frac{\text{Moles of N-Acetylglycine Obtained}}{\text{Moles of Paraformaldehyde Charged}} \times 100\%$$

To illustrate the process of the invention, the following examples are given.

Example I

A 183 ml rocking autoclave, equipped with a glass-liner, was charged with dicobalt octacarbonyl (034 g, 1.0 mmole) phenylsulfoxide (0.202 g, 1.0 mmole), paraformaldehyde (2.0 g, 66 mmole), acetamide (5.9 g, 100 mmole) and ethyl acetate (15 g). The reactor was sealed and flushed with the mixture of $CO/H_2$ (1:1 molar ratio). The system was pressured with $CO/H_2$ (1:1) to 83 bar and then pressured with pure CO to 159 bar, resulting in a ca. 3:1 molar ratio of CO to $H_2$. The autoclave was heated to 120°C and held at this temperature for ~2 hours. The maximum pressure of the reactor was 200 bar during the run. After the designated reaction time, the system was cooled to room temperature and the excess gas was vented. The resulting product materials were filtered. The solid (6.5 g) and liquid (17.9 g, brown color) materials were recovered. The H-nmr analysis showed two products, N-acetylglycine (I) and bisamidal (II) in the solid material.

The molar ratio of N-acetylglycine(I) and bisamidal(II) in the product solid is 17.6:1.0.

The yield of N-acetylglycine(I) basis paraformaldehyde charged is 68 mole%. The yield of bisamidal(II) is 4%.

The relative selectivity for (I) and (II) is 94 mole% versus 6 moles%.

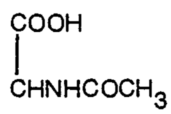 (I) 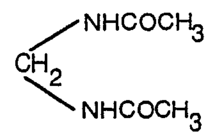 (II)

The liquid product fraction was found to contain 5250 ppm soluble cobalt. This represents an Ca. 81% recovery of cobalt in solution basis $Co_2(CO)_8$ charged.

It is of note that no significant amounts of compounds (I) and (II) can be found in the liquid product fraction. This synthesis procedure therefore allows for a relatively easy separation of N-acetylglycine product solid(I) from the soluble cobalt-containing catalyst fraction.

Example II—X

In Examples II—X the same procedure was followed as was used in Example I but with the following differences in cobalt catalyst composition, solvent composition, gas composition and operating conditions. It may be noted that:

The data for Example I, using the cobalt carbonyl-diphenylsulfoxide catalyst combination, show higher product selectivity to N-acetylglycine(I) and higher cobalt recovery in solution than for those cases (Examples II and III) with no added sulfoxide ligand promoter.

The presence of a hydrogen gas component is proven to be important basis both N-acetylglycine product selectivity and cobalt recovery in solution (cf. Examples I and IV).

Both ester and ether type solvents are effective.

TABLE I

N-acetylglycine synthesis via paraformaldehyde carbonylation

| Example[a] | Added Sulfoxide Promoter | Solvent | Reaction Conditions | Product Ratio (Mole%) I | Product Ratio (Mole%) II | Solid Product (g) I + II | Yield of I (Mole%) | Cobalt Recovery[b] (ppm) |
|---|---|---|---|---|---|---|---|---|
| I | $Ph_2SO$ (0.202g) | EtOAc (15g) | $CO/H_2$=Ca. 3:1<br>200 bar<br>120°C, 2 hr. | 94 | 6 | 6.5 | 68 | 5250 |
| II | None | EtOAc (15g) | $CO/H_2$=Ca. 3:1<br>184 bar<br>120°C, 2 hr. | 77 | 23 | 6.5 | 64 | <5 |
| III | None | EtOAc (15g) | $CO/H_2$=Ca. 3:1<br>200 bar<br>120°C, 2 hr. | 80 | 20 | 5.7 | 58 | N.D.[c] |
| IV | $Ph_2SO$ (0.202g) | EtOAc (15g) | CO Only<br>200 bar<br>120°C, 2 hr. | 79 | 21 | 4.3 | 41 | 58 |
| V | $Ph_2SO$ (0.202g) | EtOAc (15g) | $CO/H_2$=Ca. 3:1<br>200 bar<br>124—138°C, 1 hr. | 76 | 24 | 5.4 | 52 | N.D.[c] |
| VI | $Ph_2SO$ (0.404g) | p-Dioxane (15g) | $CO/H_2$=Ca. 3:1<br>207 bar<br>119—127°C, 1 hr. | 53 | 47 | 4.6 | 30 | 4660 |
| VII | $Ph_2SO$ (0.202g) | MeOAc (20g) | $CO/H_2$=Ca. 3:1<br>203 bar<br>120°C, 1 hr. | 100 | 0 | 5.1 | 59 | 3320 |
| VIII | $Ph_2SO$ (0.606g) | MeOAc (15g) | $CO/H_2$=Ca. 3:1<br>210 bar<br>120°C, 2 hr. | 93 | 7 | 5.6 | 60 | 1820 |

[a] Charge in Each Run: $CO_2(CO)_8$, 0.34g, 1.0 mmole: paraformaldehyde, 2.0g, 67 mmole; acetamide, 5.9, 100 mmole.
[b] Cobalt recovery in solution.
[c] N.D. = Not Determined.

**Claims**

1. A process for producing N-acyl-α-amino acids which comprises reacting an aldehyde, carbon monoxide, hydrogen and an amide in a substantially inert solvent with a catalyst comprising a cobalt-containing compound promoted by a ligand characterized in that, the ligand contains one or more sulfoxide groups and the process is carried out at a pressure of at least 34 bar and a temperature of at least 25°C.

2. The process of Claim 1 wherein the aldehyde is paraformaldehyde.

3. The process of Claim 1 or Claim 2 wherein the amide is acetamide.

4. The process of any of the preceding claims wherein the cobalt-containing compound is selected from cobalt(II) acetate, cobalt(II) chloride, cobalt(II) bromide, and preferably dicobalt octacarbonyl.

5. The process of any of the preceding claims wherein the sulfoxide-containing ligand used to promote the cobalt catalyst is diphenyl sulfoxide.

6. The process of any of the preceding claims wherein the solvent is selected from methyl acetate, p-dioxane, and preferably ethyl acetate.

7. The process of any of the preceding claims wherein the pressure is from 69 bar to 241 bar.

8. The process of any of the preceding claims wherein the temperature is from 80°C to 150°C.

9. The process of any of the preceding claims wherein the hydrogen to carbon monoxide ratio is in the range 20:1 to 1:20 and preferably in the range 1:1 to 1:5.

10. The process of any of the preceding claims for producing N-acetylglycine.

**Patentansprüche**

1. Verfahren zur Herstellung von n-Acyl-alpha-amino-säuren umfassend die Reaktion eines Aldehyds, Kohlenmonoxid, Wasserstoff und eines Amids in einem im wesentlichen innerten Lösungsmittel mit einem Katalysator, der eine durch einen Liganten aktivierte Kobalt enhaltende Verbindung enthält oder daraus besteht, dadurch gekennzeichnet, daß de Ligant eine oder mehrere Sulfoxid-Gruppen enthält und das Verfahren bei einem Druck von mindestens 34 bar und einer Temperatur von mindestens 25°C durchgeführt wird.

2. Vefahren nach Anspruch 1, worin der Aldéhyd Paraformaldehyd ist.

3. Verfahren nach Anspruch 1 oder Anspruch 2 worin das Amid Acetamid ist.

4. Verfahren nach einem der vorhergehenden Ansprüche, worin die Kobalt enthaltende Verbindung Kobalt-(II)-acetat, Kobalt-(II)-chlorid oder Kobalt-(II)-bromid und vorzugsweise Dikobalt-octracarbonyl ist.

5. Verfahren nach einem der vorhergehenden Ansprüche, worin der Sulfoxid enthaltende Ligand, welcher zur Aktivierung des Kobalt Katalysators eingesetzt wird, ein Diphenylsulfoxid ist.

6. Verfahren nach einem der vorhergehenden Ansprüche, worin das Lösungsmittel Methylacetat oder para-Dioxan und vorzugsweise Ethylacetat ist.

7. Verfahren nach einem der vorhergehenden Ansprüche, worin der Druck von 69 bis 241 bar beträgt.

8. Verfahren nach einem der vorhergehenden Ansprüche, worin die Temperatur 80 bis 150°C beträgt.

9. Verfahren nach einem der vorhergehenden Ansprüche, worin das Verhältnis Wasserstoff zu Kohlenmonoxid im Bereich von 20:1 bis 1:20 und vorzugsweise im Bereich von 1:1 bis 1:5 ist.

10. Verfahren nach einem der vorhergehenden Ansprüche, zur Herstellung von N-Acetylglycin.

**Revendications**

1. Procédé de production de N-acyl-α-aminoacides, qui consiste à faire réagir un aldéhyde, de l'oxyde de carbone, de l'hydrogène et un amide dans un solvant principalement inerte avec un catalyseur comprenant un composé contenant du cobalt activé par un ligand, caractérisé en ce que ligand contient un ou plusieurs groupes sulfoxyde et en ce qu'il est mis en oeuvre à une pression d'au moins 34 bars et à une température d'au moins 25°C.

2. Procédé suivant la revendication 1, dans lequel l'aldéhyde est le paraformaldéhyde.

3. Procédé suivant la revendication 1 ou la revendication 2, dans lequel l'amide est l'acétamide.

4. Procédé suivant l'une quelconque des revendications précédentes, dans lequel le composé contenant du cobalt est choisi entre l'acétate de cobalt (II), le chlorure de cobalt (II), le bromure de cobalt (II) et de préférence le dicobalt-octacarbonyle.

5. Procédé suivant l'une quelconque des revendications précédentes, dans lequel le ligand contenant un ou plusieurs groupes sulfoxyde utilisé pour activer le catalyseur au cobalt est le diphénylsulfoxyde.

6. Procédé suivant l'une quelconque des revendications précédentes, dans lequel le solvant est choisi entre l'acétate de méthyle, le p-dioxanne et de préférence l'acétate d'éthyle.

7. Procédé suivant l'une quelconque des revendications précédentes, dans lequel le pression va de 69 bars à 241 bars.

8. Procédé suivant l'une quelconque des revendications précédentes, dans lequel le température va de 80°C à 150°C.

9. Procédé suivant l'une quelconque des revendications précédentes, dans lequel le rapport de l'hydrogène à l'oxyde de carbone se situe dans intervalle de 20:1 à 1:20 et de préférence dans l'intervalle de 1:1 à 1:5.

10. Procédé suivant l'une quelconque des revendications précédentes, pour la production de N-acétylglycine.